Europäisches Patentamt

(19) European Patent Office

Office européen des brevets

(11) Veröffentlichungsnummer: **0 169 252**
**B1**

(12) # EUROPÄISCHE PATENTSCHRIFT

(45) Veröffentlichungstag der Patentschrift:
**29.11.89**

(51) Int. Cl.⁴: **G 01 N 33/44, G 01 N 11/00**

(21) Anmeldenummer: **84108401.5**

(22) Anmeldetag: **17.07.84**

(54) **Verfahren zur Viskositätsmessung von Kunstharzen und Vorrichtung zur Durchführung des Verfahrens.**

(43) Veröffentlichungstag der Anmeldung:
**29.01.86 Patentblatt 86/5**

(45) Bekanntmachung des Hinweises auf die Patenterteilung:
**29.11.89 Patentblatt 89/48**

(84) Benannte Vertragsstaaten:
**AT BE CH DE FR GB IT LI LU NL SE**

(56) Entgegenhaltungen:
**EP-B- 0 019 052**
**DE-A- 3 326 691**
**FR-A- 1 302 821**
**FR-A- 2 287 699**
**FR-A- 2 452 703**
**US-A- 3 493 345**

(73) Patentinhaber: **RHE Händel Engineering GmbH, Alte Heerstrasse 34, D-5205 St. Augustin 1, Niederpleis (DE)**

(72) Erfinder: **Händel, Max Dieter, Alte Siegburger Strasse 12, D-5204 Lohmar 1, Helde (DE)**

(74) Vertreter: **Köhne, Friedrich, Dipl.-Ing., Rondorferstrasse 5a, D-5000 Köln 51 (Marienburg) (DE)**

# Beschreibung

## Technisches Gebiet

Die Erfindung bezieht sich auf ein Verfahren zur Viskositätsmessung von Kunstharzen während des Prozessablaufes bei der Herstellung der Kunstharze in einem Reaktor, wobei ein kleiner Teilstrom des Kunstharzes bei einer Temperatur von etwa 250°C kontinuierlich aus dem Reaktor abgezogen und dessen Viskosität kontinuierlich gemessen wird und wobei die kontinuierlich gemessene Viskosität angezeigt, registriert und als prozesstechnische Kenngrösse zur automatischen Prozesssteuerung des Reaktors, insbesondere zum Abbruch des Prozesses durch Einleitung der Kühlung des Reaktors, verwendet wird.

## Stand der Technik

Bei der Herstellung von Kunstharzen muss während des Fabrikationsprozesses die Viskosität als massgebende Kenngrösse für den Prozessablauf ständig kontrolliert werden. Das übliche Verfahren hierfür, das z.B. in der EP-B 0 019 052 beschrieben ist, ist heute die Probeentnahme aus dem Reaktor. Diese Probe wird anschliessend im Labor untersucht, und die Ergebnisse entscheiden über den weiteren Fortgang des Prozesses. Die Probeentnahme und Feststellung der Viskosität im Labor erfordert im allgemeinen einen Zeitaufwand von einer Viertel- bis Dreiviertelstunde. Da insbesondere gegen Ende des Prozesses die Viskosität immer schneller ansteigt, kommt es hier durch den erforderlichen Zeitaufwand für die Viskositätsbestimmung zur Überschreitung der Lieferspezifikationswerte.

Hinzu kommt ein weiteres Problem, nämlich dass zwischen der Schmelzfluss-Viskosität, d.h. der Viskosität des Produktes im Reaktor und der Viskosität des Harzes entsprechend der Lieferspezifikation, also dem Endprodukt des Kunstharzes, keine eindeutige Beziehung besteht. Dies ist insbesondere der Fall, wenn dem Kunstharz ein Lösungsmittel beigegeben wird. Solange es sich um die Herstellung makromolekularer Stoffe handelt, die aus scharf definierten chemischen Rohstoffen aufgebaut sind, ist dieses Problem geringfügig. Die Herstellung ungesättigter Polyester ist dafür ein Beispiel. Werden jedoch Rohstoffe, wie z.B. Öle und Fette zur Herstellung des Kunstharz-Produktes verwendet, gibt es erhebliche Probleme. Hier fehlt fast in allen Fällen eine eindeutige Viskositätsbeziehung zwischen der Schmelzfluss-Viskosität und der Lösungsmittel-Viskosität bzw. der Viskosität des Endprodukts. Hinzu kommt, dass geringe Abweichungen in der Lösungsmittelzusammensetzung zu weiteren Abweichungen im Endprodukt führen können.

Ferner ist an sich bekannt, dass der Feststoffgehalt von Kunstharz-Lösungen im Normalfall zwischen 50% und 70% liegt. Dementsprechend wird bei der Viskositätsbestimmung im Labor nach der Probeentnahme das Kunstharz in gleichem Verhältnis verdünnt und danach bei einer festgelegten Temperatur die Viskosität bestimmt. Hierbei wird die Probereihe solange fortgesetzt, bis die gewünschte Endspezifikation erreicht ist. Danach wird der Prozess im Reaktor in den meisten Fällen durch Kühlen abgebrochen. Infolge der oft wiederholten Entnahme von Proben, die dann einzeln behandelt und für die dann jeweils die Viskosität bestimmt werden muss, kommt es immer wieder vor bzw. es lässt sich kaum vermeiden, dass der Endpunkt des Prozessablaufs überschritten bzw. unterschritten wird, was zu Viskositätsabweichungen zur gewünschten Viskosität des Endprodukts führt.

Ein zu Anfang angegebenes Verfahren ist aus dem Dokument FR-A 1 302 821 bekannt. Es wurde hier bereits das zuvor erläuterte Problem erkannt, dass es für die Viskositätsmessung nicht ausreicht, in längeren Zeitabständen jeweils eine Probe aus dem Reaktor zu entnehmen und im Labor zu untersuchen. Es wird daher ein Weg aufgezeigt, wie die Probeentnahme kontinuierlich durchgeführt werden kann und wie auch die Messungen kontinuierlich vorgenommen werden können. Hierbei werden die Proben unter einen erheblichen Druck gesetzt. Dieser Druck ist erforderlich, weil die gesamte vorgeschlagene Messvorrichtung darauf beruht, dass die jeweilige Probe durch eine mit einer Öffnung versehene Platte hindurchgepresst wird. Der jeweilige Druckaufbau benötigt eine gewisse Zeit, so dass von der Probeentnahme bis zur Anzeige des Drucks auf einem Anzeigegerät etwa zehn Minuten verstreichen. Des weiteren werden die Messungen zwangsweise in einem Temperaturbereich zwischen 150°C und 250°C vorgenommen. Für Polyäthylen ist ein Temperaturbereich von 170°C bis 200°C und für Polypropylen 210°C bis 240°C angegeben, wobei jedoch die Proben jeweils auf eine konstante Temperatur von beispielsweise 190°C gebracht werden. Bei dieser Verfahrensweise erhält man Messergebnisse, die in der Praxis viel zu ungenau sind und die vor allem keinerlei Rückschlüsse auf die Viskosität zulassen, die das Endprodukt aufweist, wenn man zu dem jeweiligen Messzeitpunkt den Prozessablauf im Reaktor unterbricht.

Eine weitere Messmethode zur Viskositätsmessung ist aus dem Dokument US-A 3 493 345 bekannt. Hierbei wird von der Erkenntnis ausgegangen, dass Änderungen in der Viskosität entsprechende Änderungen der Leistung eines Motors verursachen, der eine Förderpumpe antreibt. Zur Messung, die kontinuierlich erfolgt, wird ein sensibles Hall-Effekt-Wattmeter vorgeschlagen.

In einem weiteren Dokument FR-A 2 287 699 wird eine besondere Bauart eines Viskosimeters beschrieben. Danach wird eine Probe, deren Viskosität gemessen und durch einen Rührer beeinflusst werden soll, in ein Gefäss eingegeben. Ein Elektromotor treibt mit konstanter Geschwindigkeit den Drehbehälter an, welcher im Innern ein Potentiometer aufweist. Bekannt ist hierbei ferner, das Gefäss, welches eine kleine Menge Kunstharz enthält, auf eine bestimmte Temperatur zu regeln.

Bekannt sind schliesslich noch Durchlauf-Viskosimeter als solche, welche in der Praxis zur direkten Viskositätsmessung von Flüssigkeiten Verwendung finden.

Darstellung der Erfindung

Ausgehend von den zu Anfang angegebenen Verfahren liegt der Erfindung die Aufgabe zugrunde, ein solches Verfahren zur Viskositätsmessung zu schaffen, dass jederzeit und ohne Zeitverzug Messergebnisse gewonnen werden, die denjenigen entsprechen, die das Kunstharz nach Lösungsmitteluntermischung haben würde, wenn zu dem betreffenden Zeitpunkt die Reaktion im Kessel abgebrochen würde.

Die gestellte Aufgabe wird erfindungsgemäss dadurch gelöst, dass dem Teilstrom eine dem Endprodukt entsprechende Lösungsmittelmenge kontinuierlich zugeführt und mit diesem gemischt wird, dass diese Mischung anschliessend auf eine Temperatur von etwa 50°C abgekühlt wird, so dass eine Übereinstimmung mit dem Endprodukt gegeben ist, dass die abgekühlte Mischung kontinuierlich einem Durchlauf-Viskosimeter zugeführt wird.

Eine vorteilhafte Weiterentwicklung des Verfahrens ergibt sich aus Anspruch 2.

Die Erfindung betrifft ferner eine Vorrichtung zur Durchführung des vorerläuterten Verfahrens mit einem Reaktor sowie einer Abzweigleitung für einen kleinen Teilstrom des Kunstharz-Reaktorinhalts und mit einer Förderpumpe in einer Abzweigleitung. Die Vorrichtung ist dadurch gekennzeichnet, dass die Abzweigleitung einen in Form eines Wärmeaustauschers ausgebildeten Kühler aufweist, der im Betrieb auf etwa 50°C abkühlt, dass an die Abzweigleitung zwischen der Förderpumpe und dem Kühler eine Lösungsmittelzuleitung mit einer Dosierpumpe angeschlossen ist, dass die Abzweigleitung in Strömungsrichtung nach dem Kühler an ein Durchlauf-Viskosimeter angeschlossen ist, und dass der Kühler mit einer Temperaturregelungsvorrichtung ausgestattet ist.

Weitere Ausgestaltungen der Vorrichtung ergeben sich aus den Unteransprüchen 4 bis 8.

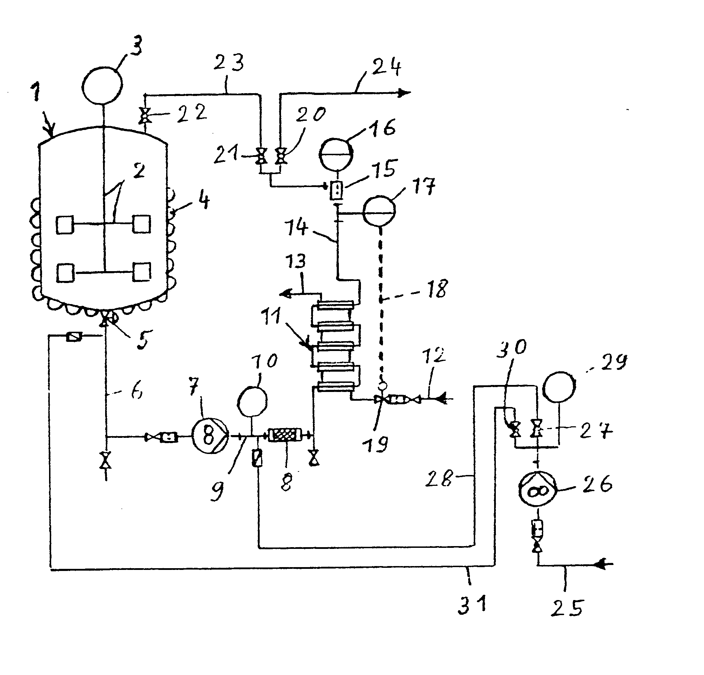

Beschreibung der Zeichnung

In der Zeichnung ist ein Ausführungsbeispiel der erfindungsgemässen Vorrichtung bzw. Anlage im Schema dargestellt. Anhand dieser Zeichnung ist die Erfindung nachfolgend näher erläutert.

Die erfindungsgemässe Vorrichtung weist einen Reaktor 1 bzw. einen Reaktionskessel auf, der in an sich bekannter Weise mit einem Rührwerk 2 versehen ist, welches mittels eines Motors 3 angetrieben wird. Der Mantel und der Boden des Reaktors ist mit einer vereinfacht gezeichneten Heiz- und Kühleinrichtung 4 ausgestattet, die ebenfalls an sich bekannt ist. Es versteht sich, dass der Reaktor auch mit nicht gezeichneten Vorrichtungen zum Einfüllen und zum Entleeren des Kunstharz-Reaktorinhalts versehen ist. Im unteren Bereich des Reaktors ist über ein Ventil eine Abzweigleitung 6 angeschlossen, die zu einer Förderpumpe 7 führt. Die Förderpumpe dient dazu, aus dem Reaktor einen kleinen Teilstrom des Kunstharz-Reaktorinhaltes kontinuierlich abzuziehen und über eine Verbindungsleitung 9 in einen als Kühler dienenden Wärmeaustauscher 11 zu führen. In diesem Wärmeaustauscher 11 wird der Teilstrom des jeweiligen Produktes auf eine konstante Temperatur abgekühlt, die von der jeweiligen Art des herzustellenden Kunstharzes abhängig ist, z. B. erfolgt die Abkühlung von etwa 250°C auf etwa 50°C. Der Wärmeaustauscher 11 besitzt einen Kühlwasservorlauf 12 und einen Kühlwasserrücklauf 13. Im Kühlwasservorlauf 12 befindet sich ein Regelventil 19, durch welches die Abkühlung auf konstante Temperatur geregelt werden kann, und zwar beispielsweise abhängig von der Messtelle des Temperaturmess- und -anzeigegerätes 17 über eine symbolisch gestrichelt gezeichnete Steuerleitung 18.

Durch Wirkung der Förderpumpe 7 gelangt der Teilstrom im abgekühlten Zustand über die Leitung 14 zu einem Viskosimeter 15 mit Messtopf. Das Viskosimeter bzw. das Viskositätsmessgerät besitzt geeignete Mess- und Anzeigegeräte, die vereinfacht dargestellt und mit dem Bezugszeichen 16 versehen sind. Von wesentlicher Bedeutung ist, dass mittels des Viskosimeters 15 die Viskosität des Teilstromes des jeweiligen Kunstharz-Reaktorinhaltes kontinuierlich gemessen, angezeigt, registiriert und als prozess-technische Kenngrösse zur automatischen Prozesssteuerung des Reaktors verwendet wird. Wenn insbesondere die Viskosität entsprechend der Lieferspezifikation festgestellt wird, kann der Prozess im Reaktor durch Einleitung der Kühlung des Reaktors abgebrochen werden. Nach dieser kontinuierlichen Viskositätsmessung kann der Teilstrom über Ventil 21, Leitung 23 und Ventil 22 wieder zum Reaktor 1 zurückgeführt werden. Insgesamt ergibt sich dadurch ein By-pass zu dem Reaktor.

Die Förderpumpe 7 ist vorteilhafterweise stufenlos regelbar eingerichtet, so dass ein gewünschter konstanter Teilstrom einstellbar ist.

Im allgemeinen wird dem Kunstharz in der Praxis Lösungsmittel beigegeben. Aus diesem Grunde wird auch dem abgezweigten Teilstrom ein Lösungsmittel kontinuierlich und in einem solchen bestimmten Verhältnis zugeführt und mit diesem gemischt, dass eine Übereinstimmung mit dem Endprodukt gegeben ist. Nach dem Vermischen des Teilstroms mit dem Lösungsmittel wird anschliessend die vorerläuterte Abkühlung in dem Wärmeaustauscher 11 vorgenommen. In Abhängigkeit von der Art des Kunstharzes wird soviel Lösungsmittel zugeführt, dass der Feststoffgehalt der Kunstharz-Lösung im Normalfall 50% bis 70% beträgt. Die Temperatur und das Mischungsverhältnis bzw. der Feststoffgehalt des Teilstroms werden kontinuierlich überwacht und ggfs. geregelt. Während der Kunstharz-Teilstrom ohne Lösungsmittel, wie oben erläutert wurde, nach Durchlauf des Viskosimeters zum Reaktor zurückgeführt wird, kann man die gleiche Rückführung zum Reaktor auch vornehmen, wenn der Kunstharz-Teilstrom mit Lösungsmittel gemischt ist, denn der verhältnismässig kleine Lösungsmittelanteil, der dadurch in den Reaktor gelangt, hat praktisch keinen Einfluss auf den Prozessablauf im Reaktor; jedoch kann man in diesem Falle auch den mit Lösungsmittel vermischten Teilstrom über das Ventil 20 und die Leitung 24 zu einem Verdünnungsbehälter oder einem Auffangbehälter zuleiten.

Vorrichtungsmässig wird, wie die Zeichnung wiedergibt, an die Abzweigleitung 9 zwischen der Förderpumpe 7 und dem Wärmeaustauscher 11 eine Lösungsmittelzuleitung mit den Leitungen 25 und 28 angeschlossen. Diese Lösungsmittelzuleitung ist mit einer eigenen Dosierpumpe 26 ausgestattet. Um eine gute Durchmischung des Teilstromes aus Kunstharz und Lösungsmittel herbeizuführen, weist die Abzweigleitung 6, 9, 14 einen stationären bzw. statischen Mischer 8 auf. Dieser Mischer 8 kann, wie in der Zeichnung dargestellt, als Einzelgerät im Bereich zwischen der Anschlusstelle der Lösungsmittelzuleitung 28 und dem Wärmeaustauscher 11 vorgesehen sein. Eine andere konstruktive vorteilhafte Lösung besteht darin, dass der Mischer als Verdrängerkörper innerhalb des Wärmeaustauschers 11 ausgebildet ist.

Wie oben angedeutet wurde, ist der Wärmeaustauscher 11 mit einer Temperaturregelungsvorrichtung ausgestattet, die vereinfacht gezeichnet und mit den Bezugszeichen 17, 18 und 19 versehen ist. Es können noch an geeigneten Stellen Druckmessgeräte 10 und 29 sowie Ventile bzw. Absperrhähne vorgesehen werden. Normalerweise ist das Ventil 27 geöffnet, so dass kontinuierlich ein bestimmter Lösungsmittelstrom durch die Dosierpumpe 26 zugeleitet werden kann. Daneben ist vorteilhafterweise noch eine Spülleitung 31 mit einem Ventil 30 vorhanden, so dass man unter entsprechendem Öffnen und Schliessen der jeweiligen Ventile das gesamte Leitungssystem mit Lösungsmittel oder einem anderen geeigneten Mittel durchspülen und reinigen kann.

Es sei hier noch einmal zusammenfassend festgestellt, dass es bei der Erfindung besonders darauf ankommt, eine schnelle bzw. kontinuierliche Viskositätsbestimmung durchzuführen. Das erfindungsgemässe kontinuierliche Viskositäts-Messverfahren misst nicht im Reaktor die Schmelzfluss-Viskosität mit der dabei zu erwartenden Problematik, die weiter oben näher erläutert worden ist, sondern führt die Viskositätsmessung vorteilhafterweise in einem By-pass zum Reaktor durch, wobei der Feststoffgehalt, nämlich Kunstharz aus dem Reaktor, und Lösungsmittel in einem ganz bestimmten Verhältnis zueinander gemischt werden, so dass eine Übereinstimmung mit dem Endprodukt in Lieferform. Weiterhin wird die Mischung im By-pass wie erläutert auf eine konstante Temperatur gebracht, wobei ständig die Temperatur, das Mischungsverhältnis bzw. der Feststoffgehalt überwacht wird. Mit dem Durchlauf-Viskosimeter wird in dem By-pass die Viskosität kontinuierlich gemessen, angezeigt, registriert und als prozesstechnische Kenngrösse verwendet.

## Patentansprüche

1. Verfahren zur Viskositätsmessung von Kunstharzen während des Prozessablaufes bei der Herstellung der Kunstharze in einem Reaktor, wobei ein kleiner Teilstrom des Kunstharzes bei einer Temperatur von etwa 250°C kontinuierlich aus dem Reaktor abgezogen und dessen Viskosität kontinuierlich gemessen wird und wobei die kontinuierlich gemessene Viskosität angezeigt, registriert und als prozesstechnische Kenngrösse zur automatischen Prozesssteuerung des Reaktors, insbesondere zum Abbruch des Prozesses durch Einleitung der Kühlung des Reaktors, verwendet wird, dadurch gekennzeichnet, dass dem Teilstrom eine dem Endprodukt entsprechende Lösungsmittelmenge kontinuierlich zugeführt und mit diesem gemischt wird, dass diese Mischung anschliessend auf eine Temperatur von etwa 50°C abgekühlt wird, so dass eine Übereinstimmung mit dem Endprodukt gegeben ist, dass die abgekühlte Mischung kontinuierlich einem Durchlauf-Viskosimeter zugeführt wird.

2. Verfahren nach Anspruch 1, dadurch gekennzeichnet, dass der mit Lösungsmitteln gemischte Teilstrom vom Durchlauf-Vikosimeter wahlweise zum Reaktor zurückgeführt oder einem Verdünnungsbehälter oder einem Auffangbehälter zugeleitet wird.

3. Vorrichtung zur Durchführung des Verfahrens nach Anspruch 1 oder 2, mit einem Reaktor (1) sowie einer Abzweigleitung (6, 9, 14) für einen kleinen Teilstrom des Kunstharz-Reaktorinhalts und mit einer Förderpumpe (7) in der Abzweigleitung, dadurch gekennzeichnet, dass die Abzweigleitung (6, 9, 14) einen in Form eines Wärmeaustauschers ausgebildeten Kühler aufweist, der im Betrieb auf etwa 50°C abkühlt, dass an die Abzweigleitung (9) zwischen der Förderpumpe (7) und dem Kühler (11) eine Lösungsmittelzuleitung (25, 28) mit einer Dosierpumpe (26) angeschlossen ist, dass die Abzweigleitung (6, 9, 14) in Strömungsrichtung nach dem Kühler (11) an ein Durchlauf-Viskosimeter (15) angeschlossen ist, und dass der Kühler (11) mit einer Temperaturregelungsvorrichtung (17, 18, 19) ausgestattet ist.

4. Vorrichtung nach Anspruch 3, dadurch gekennzeichnet, dass die Förderpumpe (7) stufenlos regelbar eingerichtet ist, so dass ein gewünschter konstanter Teilstrom einstellbar ist.

5. Vorrichtung nach Anspruch 3 oder 4, dadurch gekennzeichnet, dass die Abzweigleitung (6, 9, 14) einen stationären Mischer (8) für den Teilstrom und das Lösungsmittel aufweist.

6. Vorrichtung nach Anspruch 5, dadurch gekennzeichnet, dass der Mischer (8) im Bereich zwischen der Anschlussstelle der Lösungsmittelzuleitung (28) und dem Kühler (11) vorgesehen ist.

7. Vorrichtung nach Anspruch 5, dadurch gekennzeichnet, dass der Mischer als Verdrängerkörper innerhalb des Wärmeaustauschers (11) ausgebildet ist.

8. Vorrichtung nach einem der Ansprüche 3 bis 7, dadurch gekennzeichnet, dass an das Durchlauf-Viskosimeter (15) eine Rückführleitung (23) zu dem Reaktor (1) und eine Ablassleitung (24) zu einem Verdünnungsbehälter oder einem Auffangbehälter angeschlossen sind, die mittels Ventilen (20, 21, 22) wahlweise öffenbar oder schliessbar sind.

## Claims

1. Method for measuring the viscosity of synthetic resins during the process when manufacturing synthetic resins in a reactor, a small partial stream of synthetic resin at a temperature of approximately 250 °C being withdrawn continuously from the reactor and its viscosity being measured continuously, the continuously measured viscosity being indicated, recorded and used as a process engineering characteristic for the automatic process control of the reactor, in particular for discontinuing the process by initiating cooling of the reactor, characterised in that a quantity of solvent corresponding to the finished product is supplied continuously to the partial stream and mixed therewith, that this mixture is then cooled to a temperature of approximately 50°C, so that there is correspondence with the finished product, that the cooled mixture is supplied continuously to a throughflow viscosimeter.

2. Method according to Claim 1, caracterised in that the partial stream mixed with solvents is optionally returned by the throughflow viscosimeter to the reactor or is guided to a diluting container or collecting container.

3. Apparatus for carrying out the method according to Claim 1 or 2, with a reactor (1) and a branch pipe (6, 9, 14) for a small partial stream of the synthetic resin reactor contents and with a conveying pump (7) in the branch pipe, characterised in that the branch pipe (6, 9, 14) comprises a cooler constructed in the form of a heat exchanger, which during operation cools to approximately 50°C, that connected to the branch pipe (9) between the conveying pump (7) and the cooler (11) is a solvent supply pipe (25, 28) with a metering pump (26), that the branch pipe (6, 9, 14) is connected to a throughflow viscosimeter (15) in the flow direction after the cooler (11) and that the cooler (11) is equipped with a temperature-regulating device (17, 18, 19).

4. Apparatus according to Claim 3, characterised in that the conveying pump (7) is designed to be infinitely adjustable, so that a desired constant partial stream can be adjusted.

5. Apparatus according to Claim 3 or 4, characterised in that the branch pipe (6, 9, 14) comprises a stationary mixer (8) for the partial stream and the solvent.

6. Apparatus according to Claim 5, characterised in that the mixer (8) is provided in the region between the connecting point of the solvent supply pipe (28) and the cooler (11).

7. Apparatus according to Claim 5, characterised in that the mixer is constructed as a displacement member within the heat exchanger (11).

8. Apparatus according to one of Claims 3 to 7, characterised in that connected to the throughflow viscosimeter (15) is a return pipe (23) to the reactor (1) and a discharge pipe (24) to a diluting container or a collecting container, which can be optionally opened or closed by means of valves (20, 21, 22).

## Revendications

1. Procédé de mesure de viscosité de résines synthétiques pendant le déroulement du processus lors de la fabrication de la résine synthétique dans un réacteur, un petit courant partiel de la résine synthétique étant extrait de façon continue du réacteur à une température d'environ 250 °C et sa viscosité étant mesurée de façon continue, cette viscosité mesurée de façon continue étant affichée, enregistrée et utilisée comme une grandeur caractéristique technique du processus et en vue d'une commande automatique du fonctionnement du réacteur, notamment pour interrompre le processus par amorçage du refroidissement du réacteur, caractérisé en ce qu'on ajoute de façon continue et on mélange avec le courant partiel une quantité de solvant correspondant au produit final, en ce qu'on refroidit ce mélange ensuite à des températures d'environ 50°C de manière à établir une concordance avec le produit final et en ce que le mélange refroidi est introduit de façon continue dans un viscosimètre à passage d'écoulement.

2. Procédé selon la revendication 1, caractérisé en ce que le courant partiel, mélangé à des solvants, provenant du viscosimètre à passage d'écoulement est sélectivement renvoyé au réacteur ou bien canalisé jusqu'à un récipient de dilution ou un récipient collecteur.

3. Installation pour la mise en œuvre du procédé selon une des revendications 1 ou 2, comportant un réacteur (1) ainsi qu'un conduit de dérivation (6, 9, 14) pour un petit courant partiel du contenu de résine synthétique du réacteur et également une pompe de refoulement (7) placée dans le conduit de dérivation, caractérisée en ce que le conduit de dérivation (6, 9, 14) comporte un refroidisseur agencé comme un échangeur de chaleur et qui assure en service un refroidissement jusqu'à environ 50°C, en ce que le conduit de dérivation (9) situé entre la pompe de refoulement (7) et le refroidisseur (11) est relié à un conduit d'admission de solvant (25, 28) pourvu d'une pompe de dosage (26), en ce que le conduit de dérivation (6, 9, 14) est relié, dans la direction d'écoulement vers le refroidisseur (11), avec un viscosimètre à passage d'écoulement (15) et en ce que le refroidisseur (11) est équipé d'un dispositif de régulation de température (17, 18, 19).

4. Installation selon la revendication 3, caractérisée en ce que la pompe de refoulement (7) est agencée de façon à être réglable graduellement de manière à pouvoir régler un courant partiel constant désiré.

5. Installation selon une des revendications 3 ou 4, caractérisée en ce que le conduit de dérivation (6, 9, 14) comporte un mélangeur stationnaire (8) pour le courant partiel et pour le solvant.

6. Installation selon la revendication 5, caractérisé en ce que le mélangeur (8) est disposé dans une zone située entre le point de raccordement du conduit d'admission de solvant (28) et le refroidisseur (11).

7. Installation selon la revendication 5, caractérisée en ce que le mélangeur est agencé comme un corps de refoulement à l'intérieur de l'échangeur de chaleur (11).

8. Installation selon une des revendications 3 à 7, caractérisée en ce que le viscosimètre à passage d'écoulement (15) est relié à un conduit (23) de retour au réacteur (1) et à un conduit (24) de décharge dans un récipient de dilution ou un récipient collecteur, lesdits conduits pouvant être ouverts ou fermés sélectivement au moyen de vannes (20, 21, 22).